# EUROPEAN PATENT SPECIFICATION

(11) **EP 1 948 317 B1**
(45) Date of publication and mention of the grant of the patent: **16.10.2013**
(21) Application number: 06846328.0
(22) Date of filing: 16.11.2006
(51) Int. Cl.: A61Q 15/00, A61K 8/28, A61K 8/44, A61K 8/365

(54) **ANTIPERSPIRANT COMPOSITIONS**
SCHWEISSHEMMENDE ZUSAMMENSETZUNGEN
COMPOSITIONS ANTITRANSPIRANTES

(30) Priority: 16.11.2005 US 737207 P
(43) Date of publication of application: 30.07.2008
(60) Divisional application: 10197470.7
(73) Proprietor: Colgate-Palmolive Company, New York NY 10022-7499 (US)
(72) Inventor: MATTAI, Jairajh, Piscataway, NJ 08854 (US); KILPATRICK-LIVERMAN, Latonya, Princeton, NJ 08542 (US); HOLERCA, Marian, Somerset, NJ 08873 (US); TANG, Xiozhong, Cherry Hill, NJ 08003 (US)
(74) Representative: Jenkins, Peter David
(86) International application number: PCT/US2006/060987
(87) International publication number: WO 2007/059530

(56) References cited:
- EP-A1- 1 714 638
- WO-A-2004/089325
- WO-A-2005/092795
- WO-A1-00/67726
- US-A- 4 025 615
- US-A1- 2004 109 833
- US-A1- 2005 036 969

## Description

The present invention relates to antiperspirant compositions.

### BACKGROUND OF THE INVENTION

Antiperspirant compositions containing aluminum or aluminum-zirconium salts tend to exhibit polymerization of these salts over time, forming species with molecular weights ranging from about 500 to about 500,000 g/mol. In general, lower molecular weight species have greater antiperspirant effect than higher molecular weight species. Without being bound by theory, it is believed that the smaller molecules more readily and more effectively occlude sweat pores, thereby producing the desired antiperspirant effect. Thus, reducing the size of the polymers enhances the antiperspirant effect and moreover lowers the amount of antiperspirant salt that is necessary to control perspiration. The ability to reduce the amount of antiperspirant salt in a formulation without compromising efficacy would bring several advantages. Antiperspirant salts are a relatively expensive component of a typical antiperspirant formulation. It is thus very desirable to have compositions wherein antiperspirant salts are stabilized to reduce polymerization.

The extent of antiperspirant polymerization can be measured by size exclusion chromatography (SEC), also known as gel filtration chromatography (GFC). Passage of small molecules through an SEC column is retarded while large molecules pass through more rapidly. Elution time of a polymeric substance migrating through the SEC column is correlated with the size of the polymer molecules, and this relationship allows the apparent molecular weight of a polymer to be determined. In most cases, 4 to 6 well defined groups of polymerized species in aluminum or aluminum-zirconium salt compositions can be identified by SEC and are commonly known as peaks 1, 2, 3, 4, 5, and 6 (6 is not always present). Peak 1 is associated with zirconium species and so is present only for aluminum-zirconium salts. Peak 2 is not always present in the case of aluminum-zirconium salts. Peak 6 is not always present. The earlier peaks (1, 2, and 3) correspond to the larger species and the later peaks (4, 5 and 6) correspond to the smaller and more desirable species. Peaks correlate with weight average values of molecular weight for polymers, not as discrete values.

There remains a need in the art for improved antiperspirant and/or deodorant compositions. In particular, it would be desirable to provide such compositions having improved stability. It would further be desirable to provide such compositions having enhanced antiperspirant efficacy. It would still further be desirable to provide such compositions wherein formation of higher molecular weight zirconium and/or aluminum polymeric species is reduced or suppressed, particularly where such reduction or suppression leads to enhanced efficacy, for example related to more effective occlusion of sweat pores.

WO-A-2004/089325 discloses glycine-free antiperspirant salts combined with betaine. WO-A-2005/092795 discloses basic aluminum halogenide complexes. US-A-2005/0036969 discloses an aqueous alcoholic antiperspirant composition containing calcium enhanced antiperspirant salt.

### SUMMARY OF THE INVENTION

The present invention provides an antiperspirant composition according to claim 1. Preferred features are defined in the dependent claims.

There is also provided a method for controlling perspiration according to claim 12, a method for controlling odor according to claim 13, and a process for preparing the antiperspirant composition according to claim 14.

### DETAILED DESCRIPTION OF THE INVENTION

The components of antiperspirant composition may be present in free or salt form, and depending on the formulation, may be precipitated or dissolved or in the form of solvates.

The aluminum or aluminum-zirconium salts may be present for example in chloride or chlorohydrate form, *e.g.,* aluminum chloride, aluminum chlorohydrate, aluminum sequichlorohydrate, aluminum dichlorohydrate, aluminum zirconium tricchlorohydrate, aluminum zirconium tetrachlorohydrate, aluminum zirconium pentachlorohydrate, aluminum zirconium octachlorohydrate, or mixtures thereof. Optionally, these salts may form or be provided as complexes with other compounds, for example complexes with the hydroxy acids and/or quaternary ammonium acids as described, and/or amino acids, such as glycine, and/or polyhydric alcohols, *e.g.,* ethylene glycol, polyethylene glycol, propylene glycol, or polypropylene glycol. When the antiperspirant comprises an aluminum-zirconium salt, the salt may, for example, have a molar ratio of aluminum to zirconium of about 2 to about 10, for example, about 2 to about 5, or about 5 to about 10. In some embodiments comprising an aluminum-zirconium salt, the aluminum-zirconium salt may for example be an aluminum-zirconium chlorohydrate salt or complex thereof, wherein the molar ratio of (Aluminum and Zirconium) to chloro atoms is about 0.9 to about 2.1, for example, about 1.2 to about 1.8.

The betaine exists in zwitterionic form, forming an inner salt between cationic quaternary ammonium portion and the anionic carboxylic acid portion. It may however be present in salt form, e.g., acid addition salt form. For example betaine may be provided in the form of betaine hydrochloride.

A divalent metal cation may be provided in salt or oxide form, for example calcium may be provided in the form of, *e.g.,* calcium chloride or calcium oxide.

In one embodiment of the antiperspirant composition, the antiperspirant salt is an aluminum-zirconium chlorohydrate salt, wherein the metal (Aluminum and Zirconium) to chloride ratio is about 0.9 to about 2.1, the betaine to zirconium ratio is about 0.1 to about 2, and the betaine to hydroxy acid ratio is about 0.2 to about 30.

In one embodiment, the composition exhibits improved stability compared to analogous formulations lacking a hydroxy acid or lacking a hydroxy acid and a divalent cation, *e.g.,* as measured by at least one of (a) a reduction in formation of higher molecular weight polymerized aluminum and/or zirconium species as indicated at least by reduction in area of SEC peaks 2 and/or 3 and/or increase in area of SEC peaks 4 and/or 5; (b) a reduction in formation of higher molecular weight polymerized zirconium species as indicated at least by reduction in area of SEC peak 1; or (c) an increase in peak 5 as compared to peak 4. For example, compositions when analyzed by SEC as a 5% aqueous solution using conditions capable of resolving the Aluminum and/or Zirconium species into at least 4 successive peaks, in one embodiment, exhibit a ratio of the SEC area of the first major peak (peak 1, largest polymer) to the SEC area of the last major peak (usually peak 5) of less than about 1, and in another embodiment of less than about 0.25.

In one embodiment, there is provided an antiperspirant and/or deodorant formulation for topical use, comprising at least one stabilized antiperspirant compositions in combination with a cosmetically acceptable carrier. Such formulations may be as known in the art, e.g., in the form of a stick, gel, cream, liquid roll-on, or aerosol spray. Examples of formulations comprising the stabilized antiperspirant compositions of the invention are provided below.

SEC peak areas are used as indicators of aluminum and zirconium polymer size in antiperspirant salt solutions. It is believed, without being bound by theory, that enhanced antiperspirant and/or deodorant efficacy is associated with increase in peak 4 and/or peak 5 area relative to peak 2 and/or peak 3 area, and with decrease in peak 1 area, indicating a lower degree of polymerization of the aluminum and zirconium species respectively. Relative areas of peaks 1 through 5 constitute the "SEC profile", as that term is used herein, of an antiperspirant and/or deodorant composition. Stabilized compositions of the invention maintain, over a period of time, improved SEC profiles, indicating a lower degree of polymerization of the aluminum and/or zirconium species after aging, by comparison with non-stabilized compositions, for example compositions lacking a calcium salt and/or a hydroxy acid.

Peak areas can be adjusted based on an internal standard, with total aluminum peak area (sum of peaks 2 through 5) as the common denominator. This is possible as total aluminum peak area remains constant. Adjusting peak areas to an internal standard in this fashion better reflects the polymer distribution in the sample.

In the composition according to the invention, the relative amounts of components (a), (b), and (c) as described above are such that an aqueous solution of these components in the same relative amounts, at a 25% concentration of (a) + (b), following aging of the solution at 45°C for 60 days, has:
(i) an adjusted SEC peak 1 area of no more than 40%, for example no more than 20%, or no more than 10%;
(ii) an adjusted SEC peak 5 area of at least 30%, for example at least 40%; and
(iii) an adjusted SEC peak 4 area of at least 15%.
The adjusted SEC peak 4 area is optionally not more than 25%.

In certain embodiments, the aluminum or aluminum-zirconium salt of the antiperspirant compositions of the invention can be present in the final topical formulation in any suitable total amount, typically about 4% to about 35%, for example about 10% to about 25%, or about 15% to about 20%, by weight of the formulation. It will be understood that while amounts in various ranges of aluminum salts, aluminum-zirconium salts or complexes thereof are indicated herein, lesser amounts can be used to contribute to deodorant activity of deodorant products which are not classified as antiperspirants.

The antiperspirant compositions of the invention comprise a quaternary ammonium acid compound, namely an alpha-quaternary ammonium-carboxylic acid, which is betaine. Betaine is 1-carboxy-N,N,N-trimethylmethanaminium hydroxide inner salt (IUPAC nomenclature), and is zwitterionic. It occurs naturally in many foodstuffs and can also be synthesized. Though sometimes incorrectly referred to as an amino acid, it will be understood that betaine is not truly an amino acid. A "betaine component" herein can be betaine or a salt thereof. In certain embodiments, the betaine component comprises betaine, betaine hydrochloride or a mixture thereof. The betaine component may, for example, be present in the final formulation for topical use in a total amount of 0.5 to 20% for example 5% to 15%, by weight. In one embodiment, when the composition comprises an aluminum-zirconium salt, the molar ratio of betaine to zirconium may be 0.1 to 2, for example, 0.1 to 1.

The composition of the invention may also optionally comprise at least one divalent cation, *e.g.* in the form of a salt. In one embodiment, this cation is a calcium salt or oxide, *e.g.,* calcium chloride, calcium bromide, calcium nitrate, calcium acetate, calcium formate, calcium gluconate, calcium ascorbate, calcium lactate, calcium glycinate, calcium citrate, calcium carbonate, calcium oxide, calcium hydroxide, calcium phosphate, calcium phosphonate, calcium picrolonate, calcium sulfate, calcium sulfonate, calcium sulfocyanate, calcium perrhenate or mixtures thereof. In certain embodiments, the calcium salt comprises an anion having a linear or branched C₁₋₆₀ alkyl or alkenyl chain and at least one functional moiety capable of binding a calcium ion. The at least one functional moiety can be a nitrate, sulfate, sulfonate, carbonate, carbonyl, phosphate, phosphonate or hydroxyl moiety. The anion can comprise one or more heteroatoms independently selected from nitrogen, oxygen and sulfur, such heteroatoms being part of the chain and/or part of the functional moiety. In one embodiment, when the divalent cation is provided in the form of a salt, it may be present in the final formulation in a total amount of 0.2% to 10%, for example 2% to 8%, by weight. In one embodiment, the molar ratio of the divalent metal ion to the aluminum or (Aluminum and Zirconium) may be 0.02 to 1.2, for example 0.1 to 0.8.

The composition also comprises at least one alpha-hydroxy acid, and optionally further comprises at least one beta-hydroxy acid. In certain embodiments, the hydroxy acid is chosen from lactic acid, glycolic acid, lactobionic acid, carnitine, salicylic acid and mixtures thereof. In certain embodiments, the alpha-hydroxy acid has a pKₐ of 4.5 or less, 2.5 to 4.5, or 3.5 to 4.0.

In certain embodiments, the at least one alpha-hydroxy acid is present in a total amount of 0.2% to 10%, for example 0.5% to 9%, or 2% to 8%, by weight. In certain embodiments, the molar ratio of quaternary ammonium acid compound, to hydroxy acid is typically 0.2 to 30, for example, 5 to 25, or 10 to 20.

In certain embodiments, the composition has:
(i) a molar ratio of (Aluminum and Zirconium) to halo atoms in the at least one aluminum-zirconium salt or complex thereof of 0.9 to 2.1;
(ii) a molar ratio of aluminum to zirconium of 2 to 10;
(iii) a molar ratio of calcium to (Aluminum and Zirconium) of 0.02 to 1.2;
(iv) a molar ratio of betaine to zirconium of 0.1 to 2; and
(v) a molar ratio of betaine to hydroxy acid of 0.2 to 30.

Optional ingredients that can be included in an antiperspirant and/or deodorant formulation of the antiperspirant compositions of the invention include solvents; water-soluble alcohols such as C₂₋₈ alcohols including ethanol; glycols including propylene glycol, dipropylene glycol, tripropylene glycol and mixtures thereof; glycerides including mono-, di- and triglycerides; medium to long chain organic acids, alcohols and esters; surfactants including emulsifying and dispersing agents; amino acids including glycine; structurants including thickeners and gelling agents, for example polymers, silicates and silicon dioxide; emollients; fragrances; and colorants including dyes and pigments. If desired, an antiperspirant and/or deodorant agent additional to the aluminum-zirconium salt or complex thereof can be included, for example an odor reducing agent such as a sulfur precipitating agent, e.g., copper gluconate, zinc gluconate, zinc citrate, etc.

The antiperspirant compositions can be formulated into topical antiperspirant and/or deodorant formulations suitable for application to skin, illustratively a stick, a gel, a cream, a roll-on, a soft solid, a powder, a liquid, an emulsion, a suspension, a dispersion or a spray. The composition can comprise a single phase or can be a multi-phase system, for example a system comprising a polar phase and an oil phase, optionally in the form of a stable emulsion. The composition can be liquid, semi-solid or solid. The following topical formulations are provided for purposes of exemplification:

In one prophetic example, the formulation is in the form of a spray that comprises:
(a) about 4% to about 25% by weight in total of at least one aluminum-zirconium chlorohydrate salt or complex thereof;
(b) about 0.5 to about 20% by weight in total of at least one betaine component;
(c) about 0.2% to about 10% by weight in total of at least one calcium salt;
(d) about 0.2% to about 10% by weight in total of at least one hydroxy acid, including an alpha-hydroxy acid;
(e) about 35% to about 87% by weight of water;
(f) about 3% to about 7% by weight in total of at least one water soluble emollient; and
(g) about 0.5% to about 3% by weight in total of at least one cosmetically acceptable surfactant selected from cationic surfactants, nonionic surfactants, anionic surfactants, amphoteric surfactants, dimethicone copolyols, polyether ethoxylates, and mixtures thereof.

In another prophetic example, the formulation is in the form of a roll-on that comprises:
(a) about 4% to about 25% by weight in total of at least one aluminum-zirconium chlorohydrate salt or complex thereof;
(b) about 0.5 to about 20% by weight in total of at least one betaine component;
(c) about 0.2% to about 10% by weight in total of at least one calcium salt;
(d) about 0.2% to about 10% by weight in total of at least one hydroxy acid, including an alpha-hydroxy acid;
(e) about 27% to about 88% by weight of water;
(f) about 0.5% to about 3% by weight in total of at least one magnesium aluminum silicate;
(g) about 0.5% to about 10% by weight in total of at least one cosmetically acceptable surfactant selected from cationic surfactants, nonionic surfactants, anionic surfactants, amphoteric surfactants, dimethicone copolyols, polyether ethoxylates, and mixtures thereof; and
(h) 0% to about 5% by weight in total of at least one water miscible solvent.

In certain embodiments, the antiperspirant composition is present in the formulation in a polar phase, and the formulation further comprises an oil phase.

In another prophetic example, the formulation is in the form of a stick, wherein the polar phase comprises, by weight of the formulation:
(a) about 4% to about 30% in total of at least one aluminum-zirconium chlorohydrate salt or complex thereof;
(b) about 0.5% to about 20% in total of at least one betaine component;
(c) about 0.2% to about 10% in total of at least one calcium salt;
(d) about 0.2% to about 10% in total of at least one hydroxy acid, including an alpha-hydroxy acid;
(e) about 5% to about 40% in total of water, one or more water miscible solvents, or a mixture thereof; and
(f) 0% to about 5% in total of at least one cosmetically acceptable surfactant selected from cationic surfactants, nonionic surfactants, anionic surfactants, amphoteric surfactants, dimethicone copolyols, polyether ethoxylates, and mixtures thereof;
and the oil phase comprises, by weight of the formulation:
(g) about 0.5% to about 8.0% of a siloxane polyamide gelling agent;
(h) about 20% to about 60% of a volatile organic or silicone based fluid;
(i) 0% to about 20% in total of at least one cosmetic ingredient selected from C₈₋₂₂ fatty alcohols, C₁₂₋₃₆ fatty esters, C₈₋₁₈ alkyl benzoates, linear polysiloxanes, and mixtures thereof;
(j) 0% to about 10% in total of at least one cosmetically acceptable surfactant selected from cationic surfactants, nonionic surfactants, anionic surfactants, amphoteric surfactants, dimethicone copolyols, polyether ethoxylates, and mixtures thereof; and
(k) 0% to about 3% of a fragrance.

In another prophetic example, the formulation is in the form of a stick, wherein the polar phase comprises, by weight of the formulation:
(a) about 4% to about 30% in total of at least one aluminum-zirconium chlorohydrate salt or complex thereof;
(b) about 0.5 to about 20% in total of at least one betaine component;
(c) about 0.2% to about 10% in total of at least one calcium salt;
(d) about 0.2% to about 10% in total of at least one hydroxy acid, including an alpha-hydroxy acid;
(e) about 5% to about 40% in total of water, one or more water miscible solvents, or a mixture thereof; and
(f) 0% to about 5% in total of at least one cosmetically acceptable surfactant selected from cationic surfactants, nonionic surfactants, anionic surfactants, amphoteric surfactants, dimethicone copolyols, polyether ethoxylates, and mixtures thereof;
and the oil phase comprises, by weight of the formulation:
(g) about 20% to about 60% in total of one or more cosmetically acceptable solvents selected C₂₋₈ polyhydric alcohols, C₈₋₂₂ unsaturated fatty alcohols, branched and straight chain C₈₂₂ saturated fatty alcohols, and mixtures thereof;
(h) 0% to about 10% in total of at least one cosmetically acceptable surfactant selected from cationic surfactants, nonionic surfactants, anionic surfactants, amphoteric surfactants, dimethicone copolyols, polyether ethoxylates, and mixtures thereof;
(i) 0% to about 3% of a fragrance; and
(j) about 5% to about 25% by weight of a linoleic acid dimer based polyamide.

In another prophetic example, the formulation is in the form of a gel, wherein the polar phase comprises, by weight of the formulation:
(a) about 4% to about 35% in total of at least one aluminum-zirconium chlorohydrate salt or complex thereof;
(b) about 0.5 to about 20% in total of at least one betaine component;
(c) about 0.2% to about 10% in total of at least one calcium salt;
(d) about 0.2% to about 10% in total of at least one hydroxy acid, including an alpha-hydroxy acid;
(e) about 25% to about 60% of water;
(f) about 5% to about 40% in total of at least one water miscible solvent; and
(g) 0% to about 5% in total of at least one cosmetically acceptable surfactant selected from cationic surfactants, nonionic surfactants, anionic surfactants, amphoteric surfactants, dimethicone copolyols, polyether ethoxylates, and mixtures thereof;
and the oil phase comprises, by weight of the formulation:
(g) about 5% to about 20% by weight of a volatile organic fluid;
(h) about 0.5% to about 2% by weight of a dimethicone copolyol; and
(i) about 5% to about 20% by weight of a linear silicone.

In another prophetic example, the formulation is in the form of a cream, wherein the polar phase comprises, by weight of the formulation:
(a) about 4% to about 30% in total of at least one aluminum-zirconium chlorohydrate salt or complex thereof;
(b) about 0.5% to about 20% in total of at least one betaine component;
(c) about 0.2% to about 10% in total of at least one calcium salt;
(d) about 0.2% to about 10% in total of at least one hydroxy acid, including an alpha-hydroxy acid; and
(e) about 40% to about 82% of water;
and the oil phase comprises, by weight of the formulation:
(f) about 2% to about 10% of a volatile organic fluid;
(g) about 0.1% to about 3% of a monoglyceride, diglyceride, triglyceride, or mixture thereof;
(h) about 4% to about 15% in total of at least one cosmetically acceptable surfactant selected cationic surfactants, nonionic surfactants, anionic surfactants, amphoteric surfactants, dimethicone copolyols, polyether ethoxylates, and mixtures thereof; and
(i) about 3% to about 8% in total of at least one C₈₋₂₂ fatty alcohol.

In another prophetic example, the formulation is in the form of a roll-on, wherein the polar phase comprises, by weight of the formulation:
(a) about 4% to about 25% in total of at least one aluminum-zirconium chlorohydrate salt or complex thereof;
(b) about 0.5% to about 20% in total of at least one betaine component;
(c) about 0.2% to about 10% in total of at least one calcium salt;
(d) about 0.2% to about 10% in total of at least one hydroxy acid, including an alpha-hydroxy acid;
(e) about 30% to about 50% of water;
(f) about 5% to about 40% in total of at least one water miscible solvent; and
(g) 0% to about 2% in total of at least one cosmetically acceptable surfactant selected from cationic surfactants, nonionic surfactants, anionic surfactants, amphoteric surfactants, dimethicone copolyols, polyether ethoxylates, and mixtures thereof;
and the oil phase comprises, by weight of the formulation:
(h) about 20% to about 50% of a volatile organic or silicone based fluid; and
(i) about 0.5% to about 2% by weight of a dimethicone copolyol.

The antiperspirant and/or deodorant formulation can be provided in any suitable container such as an aerosol can, tube or container with a porous cap, roll-on container, bottle, container with an open end, etc.

A method of the invention for controlling perspiration comprises applying to skin an antiperspirant effective amount of a formulation of any embodiment embraced or specifically described herein.

A method of the invention for controlling odor from perspiration comprises applying to skin a deodorant effective amount of a formulation of any embodiment embraced or specifically described herein.

In a process of the invention for preparing an antiperspirant and/or deodorant composition or formulation, at least one aluminum-zirconium salt or complex thereof is mixed with at least one betaine component in an aqueous medium; and at least one calcium salt and at least one hydroxy acid are added to the resulting mixture to form the composition. Selection of particular aluminum-zirconium, betaine, calcium salt and hydroxy acid components and amounts of these components used can be made in accordance with the disclosure above.

Unless stated otherwise, all percentages of composition components given in this specification are by weight based on a total composition or formulation weight of 100%.

Unless otherwise specifically identified, in one embodiment, the ingredients for use in the compositions and formulations of the present invention are cosmetically acceptable ingredients. By "cosmetically acceptable" is meant suitable for use in a formulation for topical application to human skin. A cosmetically acceptable excipient, for example, is an excipient which is suitable for external application in the amounts and concentrations contemplated in the formulations of this invention, and includes for example excipients which are "Generally Recognized as Safe" (GRAS) by the United States Food and Drug Administration.

For purposes of this application, pKa means the pKa in dilute aqueous solution at room temperature and pressure, *e.g.,* at ca. 25°C, using standard, art-recognized measuring techniques. For acids that have more than one hydrogen capable of dissociation and so have multiple pKa values, the pKa for purposes of this application refers to the ionization equilibrium with respect to the first hydrogen dissociation step. Thus the pKa of lactic acid for purposes as defined herein would be about 3.86 and glycolic acid would be about 3.83.

The compositions and formulations as provided herein are described and claimed with reference to their ingredients, as is usual in the art. As would be evident to one skilled in the art, the ingredients may in some instances react with one another, so that the true composition of the final formulation may not correspond exactly to the ingredients listed. Thus, it should be understood that the invention extends to the product of the combination of the listed ingredients.

### EXAMPLE

Varying combinations of CaCl₂ and/or hydroxy acids (lactic acid or glycolic acid) are added to a 25% solution of zirconium-aluminum chlorohydrex with betaine (R309; herein "ZAB"). The resulting sample solution is then aged for two months at 45°C before analysis by SEC using a refractive index detector. Table 1 shows data for SEC peaks 1, 3, 4 and 5 for each sample solution. Adjusted area is calculated using total aluminum peak area (sum of peaks 2-5) as the common denominator.

**Table 1: Adjusted SEC peak areas following aging**

| Sample solution | Peak 1 | Peak 3 | Peak 4 | Peak 5 |
|---|---|---|---|---|
| ZAB (R309) | 25.74 | 52.37 | 22.44 | 25.19 |
| ZAB + lactic acid (2%) | 8.57 | 46.24 | 11.58 | 42.18 |
| ZAB + glycolic acid (2%) | 42.32 | 44.66 | 10.01 | 45.32 |
| ZAB + CaCl₂ (2%) | 31.92 | 42.02 | 32.91 | 25.07 |
| ZAB + CaCl₂ (2%) + lactic acid (2%) | 8.80 | 36.62 | 20.22 | 43.16 |
| ZAB + CaCl₂ (2%) + glycolic acid (2%) | 37.86 | 37.99 | 18.92 | 43.09 |

Addition of 2% lactic acid decreases peak 1 area from 25.74% (ZAB alone) to 8.57%, and in presence of CaCl₂ from 31.92% (ZAB + CaCl₂) to 8.80%. This indicates that lactic acid can reduce polymerization of zirconium species. Glycolic acid does not reduce peak 1 area in this study.

Addition of 2% lactic acid increases peak 5 area from 25.19% (ZAB alone) to 42.18%, and in presence of CaCl₂ from 25.07% (ZAB + CaCl₂) to 43.16%. Similarly, addition of 2% glycolic acid increases peak 5 area from 25.19% (ZAB alone) to 45.32%, and in presence of CaCl₂ from 25.07% (ZAB + CaCl₂) to 43.09%. These results indicate that hydroxy acids such as lactic acid and glycolic acid can reduce polymerization of aluminum species.

## Claims

1. An antiperspirant composition comprising a mixing product of:
(a) at least one salt chosen from at least one aluminum salt, at least one aluminum-zirconium salt, at least one aluminum salt complex, and at least one aluminum-zirconium salt complex, wherein the aluminum salt or the aluminum salt complex is at least one salt chosen from aluminum chloride, aluminum chlorohydrate, aluminum sequichlorohydrate, and aluminum dichlorohydrate;
(b) at least one cosmetically acceptable alpha-hydroxy acid; and
(c) at least one betaine chosen from 1-carboxy-N,N,N-trimethylmethanaminium hydroxide inner salt and 1-carboxy-N,N,N-trimethylmethanaminium hydroxide inner salt hydrochloride;
wherein relative amounts of components (a), (b), and (c) are such that an aqueous solution of these components in the same relative amounts, at a 25% concentration of (a) + (b), following aging of the solution at 45°C for 60 days, has an adjusted size exclusion chromatography "SEC" peak 1 area of no more than 40%, an adjusted SEC peak 5 area of at least 30%, and an adjusted SEC peak 4 area of at least 15%, each peak being adjusted based on the total aluminum peak area of the sum of peaks 2 through 5.

2. The composition of claim 1, wherein the salt is a chlorohydrate, optionally chosen from aluminum-zirconium pentachlorohydrate, aluminum-zirconium octachlorohydrate, aluminum-zirconium tetrachlorohydrate, aluminum-zirconium trichlorohydrate, and mixtures thereof.

3. The composition of claim 1, wherein the alpha-hydroxy acid is chosen from lactic acid, glycolic acid, and combinations thereof.

4. The composition of claim 1, wherein the alpha-hydroxy acid has a pKₐ of less than 4.5.

5. The composition of claim 1 further comprising a divalent metal cation.

6. The composition of claim 5, wherein the divalent metal cation is calcium and the ratio of calcium to metal (Aluminum and Zirconium) on a molar basis is 0.02 to 1.2.

7. The composition of claim 1 further comprising a calcium salt chosen from calcium chloride, calcium oxide, and mixtures thereof.

8. The composition of claim 1, wherein the aluminum or aluminum-zirconium salt comprises an aluminum-zirconium chlorohydrate salt, and wherein the metal (Aluminum and Zirconium) to chloride ratio on a molar basis for the salt is 0.9 to 2.1, optionally the betaine to zirconium ration on a molar basis is 0.1 to 2 and/or optionally the betaine to hydroxy acid ratio of a molar basis is 0.2 to 30.

9. The composition of claim 1, wherein the composition comprises less than 1% by weight of an amino acid.

10. The composition of claim 1, which when analyzed by size exclusion chromatography as a 5% aqueous solution using conditions capable of resolving the aluminum and/or zirconium species into at least 4 successive peaks, the ratio of the SEC area of the first major peak to the SEC area of the last major peak is less than 1, optionally less than 0.25.

11. The composition of any foregoing claim, wherein the SEC peak 1 area is no more than 10% and/or the SEC peak 5 area is at least 40%.

12. A method for controlling perspiration comprising applying to skin a formulation comprising an antiperspirant effective amount of the composition of claim 1.

13. A method for controlling odor from perspiration comprising applying to skin a deodorant effective amount of the composition of claim 1.

14. A process for preparing the antiperspirant composition of claim 1 comprising:
i. mixing the at least one salt with the at least one betaine in an aqueous medium; and
ii. adding to the resulting mixture the at least one alpha-hydroxy acid to form the composition.

## Patentansprüche

1. Antitranspirant-Zusammensetzung, die ein Mischungsprodukt umfasst von:
(a) mindestens einem Salz, ausgewählt aus mindestens einem Aluminiumsalz, mindestens einem Aluminium-Zirconiumsalz, mindestens einem Aluminiumsalz-Komplex und mindestens einem Aluminium-Zirkoniumsalz-Komplex, wobei aluminiumsalz oder der Aluminiumsalzkomplex mindestens ein Salz ist, das aus Aluminiumchlorid, Aluminiumchlorhydrat, Aluminiumsequichlorhydrat und Aluminiumdichlorhydrat ausgewählt ist;
(b) wenigstens einer kosmetisch verträglichen alpha-Hydroxysäure und
(c) wenigstens einem Betain, ausgewählt aus dem inneren Salz von 1-Carboxy-N,N,N-trimethylmethanaminium-hydroxid und dem inneren Salz von 1-Carboxy-N,N,N-trimethylmethanaminiumhydroxid-hydrochlorid;
wobei die relativen Mengen der Komponenten (a), (b) und (c) so gewählt sind, dass die wässrige Lösung dieser Komponenten in den gleichen relativen Mengen bei einer 25%igen Konzentration von (a)+(b), nach dem Altern der Lösung bei 45°C für 60 Tage, einen angepassten Peak-1-Bereich in der Größenausschlußchromatographie "SEC" von nicht mehr als 40%, einen angepassten Peak-5-Bereich von mindestens 30% und einen angepassten Peak-4-Bereich von mindestens 15% aufweist, wobei jeder Peak auf Basis der Gesamtaluminiumpeakfläche oder Summe der Peaks 2 bis 5 angepasst wurde.

2. Zusammensetzung nach Anspruch 1, wobei das Salz ein Chlorhydrat ist, das gegebenenfalls aus Aluminium-Zirkoniumpentachlorhydrat, Aluminium-Zirkoniumoktachlor-hydrat, Aluminium-Zirkoniumtetrachlorhydrat, Aluminium-Zirkoniumtrichlorhydrat und deren Gemischen ausgewählt ist.

3. Zusammensetzung nach Anspruch 1, alpha-Hydroxysäure aus Milchsäure, Glykolsäure und deren Gemischen ausgewählt ist.

4. Zusammensetzung nach Anspruch 1, wobei die alpha-Hydroxysäure einen pKₐ-Wert von weniger als 4,5 aufweist.

5. Zusammensetzung nach Anspruch 1, die weiterhin ein divalentes Metallkation umfasst.

6. Zusammensetzung nach Anspruch 5, wobei das divalente Metallkation Calcium ist und das Verhältnis von Calcium zu Metall (Aluminium und Zirkonium) auf molarer Basis 0,02 bis 1,2 beträgt.

7. Zusammensetzung nach Anspruch 1, die weiterhin ein Calciumsalz umfasst, das aus Calciumchlorid, Calciumoxid und deren Gemischen ausgewählt ist.

8. Zusammensetzung nach Anspruch 1, wobei das Aluminium oder Aluminium-Zirconiumsalz ein Aluminium-Zirconiumchlor-hydratsalz umfasst und das Verhältnis von Metall (Aluminium und Zirkonium) zu Chlorid auf molarer Basis für das Salz 0,9 bis 2,1 beträgt, gegebenenfalls das Verhältnis von Betain zu Zirconium auf molarer Basis 0,1 bis 2 beträgt und/oder gegebenenfalls das Verhältnis von Betain zu Hydroxysäure auf molarer Basis 0,2 bis 30 beträgt.

9. Zusammensetzung nach Anspruch 1, wobei die Zusammensetzung weniger als etwa 1 Gew.% einer Aminosäure enthält.

10. Zusammensetzung nach Anspruch 1, wobei, wenn sie durch Größenausschlußchromatographie als eine 5%ige wässrige Lösung unter Anwendung von Bedingungen analysiert wurde, die die Aluminium- und/oder Zirkonium-Spezies in wenigstens 4 aufeinanderfolgende Peaks auftrennen können, das Verhältnis des SEC-Bereichs des ersten Hauptpeaks zum SEC-Bereich des letzten Hauptpeaks weniger als 1, gegebenenfalls weniger als 0,25 ist.

11. Zusammensetzung nach einem der vorangehenden Ansprüche, wobei der SEC-Peak-1-Bereich nicht mehr als 10% und der SEC-Peak-5-Bereich mindestens 40% beträgt.

12. Verfahren zur Bekämpfung von Schweiß, bei dem eine Formulierung auf die Haut aufgetragen wird, die eine als Antitranspirant wirksame Menge der Zusammensetzung gemäß Anspruch 1 umfasst.

13. Verfahren zur Bekämpfung von Schweißgeruch, bei dem eine als Antitranspirant wirksame Menge der Zusammensetzung gemäß Anspruch 1 auf die Haut aufgetragen wird.

14. Verfahren zur Herstellung der schweißhemmenden Zusammensetzung gemäß Anspruch 1, bei dem:
i. das mindestens eine Salz mit dem mindestens einen Betain in einem wässrigen Medium gemischt wird und
ii. zu dem erhaltenen Gemisch mindestens eine alpha-Hydroxysäure zur Bildung der Zusammensetzung zugefügt wird.

## Revendications

1. Composition antitranspirante comprenant un produit de mélange de:
(a) au moins un sel choisi parmi au moins un sel d'aluminium, au moins un sel d'aluminium-zirconium, au moins un complexe de sels d'aluminium et au moins un complexe de sels d'aluminium-zirconium, dans laquelle le sel d'aluminium ou le complexe de sels d'aluminium est au moins un sel choisi parmi le chlorure d'aluminium, le chlorhydrate d'aluminium, le sesquichlorohydrate d'aluminium et le dichlorhydrate d'aluminium
(b) au moins un acide alpha-hydroxylé cosmétiquement acceptable; et
(c) au moins une bétaïne choisie parmi le sel interne d'hydroxyde de 1-carboxy-N,N,N-triméthylméthanaminium et le chlorhydrate du sel interne d'hydroxyde de 1-carboxy-N,N,N-triméthylméthanaminium ;
dans laquelle les quantités relatives des composants (a), (b) et (c) sont telles qu'une solution aqueuse de ces composants dans les mêmes relatives, à une concentration de 25 % de (a) + (b), après vieillissement de la solution à 45 °C pendant 60 jours, a une surface de pic de chromatographie d'exclusion diffusion « CED » 1 ajusté inférieure ou égale à 40 %, une surface de pic CED 5 ajusté supérieure ou égale à 30 %, et une surface de pic CED 4 ajusté d'au moins 15 %, chaque pic étant ajusté en fonction de la surface totale des pics d'aluminium de la somme des pics 2 à 5.

2. Composition selon la revendication 1, dans laquelle le sel est un chlorhydrate, optionnellement choisi parmi le pentachlorhydrate d'aluminium-zirconium, l'octachlorhydrate d'aluminium-zirconium, le tétrachlorhydrate d'aluminium-zirconium, le trichlorhydrate d'aluminium-zirconium et un quelconque mélange de ces éléments.

3. Composition selon la revendication 1, dans laquelle l'acide alpha-hydroxylé est choisi parmi l'acide lactique, l'acide glycolique et une quelconque combinaison de ces éléments.

4. Composition selon la revendication 1, dans dans laquelle l'acide alpha-hydroxylé a un pKₐ inférieur à 4,5.

5. Composition selon la revendication 1 comprenant en outre un cation de métal divalent.

6. Composition selon la revendication 5, dans dans laquelle le cation de métal divalent est du calcium et dans laquelle le rapport entre le calcium et le métal (aluminium et zirconium) sur une base molaire est de 0,02 à 1,2.

7. Composition selon la revendication 1 comprenant en outre un sel de calcium choisi parmi le chlorure de calcium, l'oxyde de calcium et un quelconque mélange de ces éléments.

8. Composition selon la revendication 1, dans laquelle le sel d'aluminium ou d'aluminium-zirconium comprend un sel de chlorhydrate d'aluminium-zirconium, et dans laquelle le rapport entre le métal (aluminium et zirconium) et le chlorure sur une base molaire pour le sel est de 0,9 à 2,1, dans dans laquelle optionnellement le rapport entre la bétaïne et le zirconium sur une base molaire est de 0,1 à 2, et/ou dans dans laquelle optionnellement le rapport entre la bétaïne et l'hydroxy-acide sur une base molaire est de 0,2 à 30.

9. Composition selon la revendication 1, dans laquelle la composition est constituée à moins de 1 % de son poids d'un acide aminé.

10. Composition selon la revendication 1, qui, lorsqu'elle est analysée par chromatographie d'exclusion diffusion sous la forme d'une solution aqueuse à 5 % en utilisant des conditions capables de séparer les espèces contenant de l'aluminium et/ou du zirconium en au moins 4 pics successifs, le rapport entre la surface CED du premier pic majeur et la surface CED du dernier pic majeur est inférieur à 1, optionnellement inférieur à 0,25.

11. Composition selon l'une quelconque des revendications précédentes, dans dans laquelle la surface du pic CED 1 n'est pas supérieure à 10 % et/ou dans dans laquelle la surface du pic CED 5 est supérieure ou égale à 40 %.

12. Procédé permettant de réduire la transpiration comprenant l'application sur la peau d'une formule comprenant une quantité efficace d'antitranspirant de la composition selon la revendication 1.

13. Procédé permettant de réduire les odeurs provoquées par la transpiration comprenant l'application sur la peau d'une quantité efficace de déodorant de la composition selon la revendication 1.

14. Procédé de préparation de la composition antitranspirante selon la revendication 1 comprenant :
i. le mélange de l'au moins un sel avec l'au moins une bétaïne dans un milieu aqueux ; et
ii. l'ajout au mélange résultant de l'au moins un acide alpha-hydroxylé pour former la composition.
